# EUROPÄISCHE PATENTSCHRIFT

(11) **EP 2 498 730 B1**
(45) Veröffentlichungstag und Bekanntmachung des Hinweises auf die Patenterteilung: **26.02.2014**
(21) Anmeldenummer: 10784973.9
(22) Anmeldetag: 12.11.2010
(51) Int. Cl.: A61F 2/68, A61F 2/64, A61F 2/66, A61F 5/01

(54) **VERFAHREN UND VORRICHTUNG ZUR STEUERUNG EINES KÜNSTLICHEN ORTHETISCHEN ODER PROTHETISCHEN GELENKES**
METHOD AND DEVICE FOR CONTROLLING AN ARTIFICIAL ORTHOTIC OR PROSTHETIC JOINT
PROCÉDÉ ET DISPOSITIF POUR COMMANDER UNE ARTICULATION ORTHÉTIQUE OU PROTHÉTIQUE ARTIFICIELLE

(30) Priorität: 13.11.2009 DE 102009052893
(43) Veröffentlichungstag der Anmeldung: 19.09.2012
(73) Patentinhaber: Otto Bock Healthcare Products GmbH, 1070 Wien (AT)
(72) Erfinder: SEYR, Martin, A-1040 Wien (AT); KAMPAS, Philipp, A-1020 Wien (AT); POP, Constantin, A-2630 Ternitz (AT)
(74) Vertreter: Stornebel, Kai
(86) Internationale Anmeldenummer: PCT/EP2010/006895
(87) Internationale Veröffentlichungsnummer: WO 2011/057794

(56) Entgegenhaltungen:
- US-A1- 2009 030 530

## Beschreibung

Die Erfindung betrifft ein Verfahren und eine Vorrichtung zur Steuerung eines künstlichen orthetischen oder prothetischen Gelenkes mit einer Widerstandseinrichtung, der zumindest ein Aktuator zugeordnet ist, über den der Beuge- und/oder Streckwiderstand in Abhängigkeit von Sensordaten verändert wird. Ein solches Verfahren und eine solche Vorrichtung sind besonders zur Steuerung von Gelenken der unteren Extremitäten, also des Hüftgelenkes, des Kniegelenkes oder des Knöchelgelenkes, geeignet. Grundsätzlich ist jedoch auch möglich, dass eine solche Vorrichtung und ein solches Verfahren zum Betreiben anderer prothetischer oder orthetischer künstlicher Gelenke genutzt wird, beispielsweise eines Ellenbogengelenkes oder eines Gelenkes in einer Prothesenhand.

Im zunehmenden Maße werden künstliche Gelenke mit Widerstandseinrichtungen versehen, die hinsichtlich ihres Flexionswiderstandes oder Extensionswiderstandes veränderbar sind. Die Veränderbarkeit der Widerstände hat den Vorteil, dass es möglich ist, den Widerstand an die unterschiedlichen Anforderungen während eines Bewegungsablaufes anzupassen. Um beispielsweise die unterschiedlichen Anforderungen während der verschiedenen Phasen eines Schrittes möglichst natürlich darzustellen oder zu unterstützen, sind steuerbare Widerstandseinrichtungen vorgesehen, die einen angepassten, veränderbaren Flexionswiderstand und Extensionswiderstand bereitstellen. Über den Flexionswiderstand wird eingestellt, wie leicht der Unterschenkelschaft oder die Unterschenkelschiene im Verhältnis zum Oberschenkelschaft oder der Oberschenkelschiene bei einer aufgebrachten Kraft nach hinten schwingt. Der Extensionswiderstand bremst die Vorwärtsbewegung des Unterschenkelschaftes oder der Unterschenkelschiene und bildet unter anderem einen Streckanschlag aus.

Aus der DE 10 2008 008 284 A1 ist ein orthopädietechnisches Kniegelenk mit einem Oberteil und einem verschwenkbar daran angeordneten Unterteil bekannt, dem mehrere Sensoren zugeordnet sind, beispielsweise ein Beugewinkelsensor, ein Beschleunigungssensor, ein Neigungssensor und/oder ein Kraftsensor. Der Extensionsanschlag wird in Abhängigkeit von ermittelten Sensordaten eingestellt.

Die DE 10 2006 021 802 A1 beschreibt eine Steuerung eines passiven Prothesenkniegelenkes mit verstellbarer Dämpfung in Flexionsrichtung zur Anpassung einer Protheseneinrichtung mit oberseitigen Anschlussmitteln und einem Verbindungselement zu einem Kunstfuß. Die Anpassung erfolgt an das Treppaufgehen, wobei ein momentenarmes Anheben des Prothesenfußes detektiert und die Flexionsdämpfung in der Anhebephase auf unterhalb eines Niveaus, das für ein Gehen in der Ebene geeignet ist, abgesenkt wird. Die Flexionsdämpfung kann in Abhängigkeit von der Veränderung des Kniewinkels und in Abhängigkeit von der auf den Unterschenkel wirkenden Axialkraft angehoben werden.

Aus der DE 10 2007 053 389 A1 ist ein Verfahren zur Steuerung eines orthopädischen Gelenkes einer unteren Extremität mit zumindest einem Freiheitsgrad mit einem verstellbaren Aktuator zur Anpassung einer orthopädischen Einrichtung, die oberseitige Anschlussmittel an Gliedmaße und ein distal zu den Anschlussmitteln gelenkig angeordnetes orthopädisches Element aufweist, an eine Situation bekannt, die von dem Gehen in der Ebene abweicht. Dabei werden mehrere Parameter der orthopädischen Einrichtung über Sensoren erfasst, die erfassten Parameter mit Kriterien verglichen, die anhand mehrerer Parameter und/oder Parameterverläufe erstellt wurden und in einer Rechnereinheit abgelegt sind, und anschließend ein Kriterium ausgewählt, das anhand der ermittelten Parameter und/oder Parameterverläufe geeignet ist. Die Bewegungswiderstände, Bewegungsumfänge, Antriebskräfte oder deren Verläufe werden in Abhängigkeit von dem gewählten Kriterium angepasst, um Sonderfunktionen zu steuern, die vom Gehen in der Ebene abweichen.

Die US RE39,961 E beschreibt eine computergesteuerte, hydraulische Widerstandseinrichtung für eine Prothese mit Drucksensoren. Sowohl auf der Hochdruckseite als auch auf der Niedrigdruckseite werden der Druck über einen federbelasteten Magneten und einen magnetisch aktuierten, elektronischen Sensor erfasst und in einem geschlossenen Regelkreis durch einen Mikro-Controller verarbeitet, um automatisch Variationen in der Vorrichtung und in der Viskosität der Hydraulikflüssigkeit zu kompensieren. Der Kompensation werden die Ergebnisse der Druckmessung zugrunde gelegt.

Die US 2009/0030530 A1 betrifft ein elektronisch gesteuertes Prothesensystem mit einem Sensor zum Ermitteln oder Quantifizieren einer operationalen Charakteristik einer prothetischen Gliedmaße, wobei der Sensor Nanomaterial aufweist. Basierend auf den Sensordaten wird ein Mikroprozessor oder ein Computersystem einen Dämpfer in einer vorgegeben Art und Weise beeinflussen. Als Informationen Kräfte, Temperaturen, Vibrationen oder andere Informationen zugrunde gelegt werden.

Im Hinblick auf künstliche Kniegelenksprothesen oder Orthesen hat sich bewährt, dass die Gelenke in der Standphase während des Gehens oder auch während des Stehens einen hohen Flexionswiderstand bieten, wobei das Gelenk nicht ganz gesperrt wird. In diesem Fall wird das langsame Beugen des Gelenks beim Stehen dadurch verhindert, dass der Kraftvektor vor der Gelenkachse liegt und das Gelenk somit in den Streckanschlag drückt. Ob der Kraftvektor vor der Gelenkachse liegt, ist durch den Aufbau der Prothese oder Orthese festgelegt.

Die Tatsache, dass das Gelenk in der oben beschriebenen Situation nicht vollständig sperrt, hat den Vorteil, dass der Nutzer noch Eingriffsmöglichkeiten in die Gelenksbewegung hat. Sollte er zum Beispiel auf einer Treppe stehen und das Gleichgewicht verlieren, würde er über ein gesperrtes Gelenk unkontrolliert stürzen, während er ein Gelenk mit hohem Widerstand mittels der Stumpfkraft noch beugen kann. Dadurch können die Folgen eines Sturzes vermindert oder das Stürzen ganz verhindert werden. Ebenfalls erleichtert der hohe Beugewiderstand das Manövrieren des Gelenkes in engen Räumen oder das Hinsetzen. Ein großer Nutzen des hohen Beugewiderstandes ist ebenfalls, dass alternierend Rampen und Treppen abwärts begangen werden können. Dabei übernimmt der Widerstand des Gelenkes die Unterstützung des Körpers und entlastet somit die kontralaterale Seite, also das erhaltene Bein. Diese Entlastung der erhaltenen Seite drückt sich direkt in der von der Widerstandseinrichtung dissipierten Energie aus, die zur einer sehr starken Erwärmung der Widerstandseinrichtung führen kann. Diese Erwärmung kann auch beim Gehen in der Ebene auftreten, wenn die Widerstandseinrichtung das Ausschwingen des Beins in der Schwungsphase auf einen gewünschten Wert bremst oder wenn der Träger das Gelenk während der Standphase gegen einen hohen Widerstand beugt und streckt. Grundsätzlich kann eine Erwärmung der Widerstandseinrichtung auch in anderen Gelenken erfolgen, beispielsweise im Knöchel- oder im Hüftgelenk oder auch in anderen Gelenkeinrichtungen, beispielsweise wenn dort hohe Belastungen auftreten.

Die Temperaturerhöhungen können zu einem veränderten Widerstandsverhalten und gegebenenfalls zu Beschädigungen von Komponenten der Gelenkeinrichtung führen.

Aufgabe der Erfindung ist es, eine Vorrichtung und ein Verfahren bereitzustellen, mit dem diese Probleme vermindert oder verhindert werden.

Erfindungsgemäß wird diese Aufgabe durch ein Verfahren mit den Merkmalen des Hauptanspruches und eine Vorrichtung mit den Merkmalen des nebengeordneten Anspruches gelöst. Vorteilhafte Ausgestaltungen und Weiterbildungen werden in den Unteransprüchen beschrieben.

Das erfindungsgemäße Verfahren zur Steuerung eines künstlichen orthetischen oder prothetischen Gelenkes mit einer Widerstandseinrichtung, der zumindest ein Aktuator zugeordnet ist, über den der Beuge- und/oder Streckwiderstand in Abhängigkeit von Sensordaten verändert wird, sieht vor, dass der Widerstand in Abhängigkeit einer gemessenen Temperatur oder von einem gemessenen Temperatursignal verändert wird. Dadurch ist es möglich, die Widerstandseinrichtung oder auch andere Komponenten des künstlichen orthetischen oder prothetischen Gelenkes vor einer zu starken Erwärmung zu schützen. Eine Erwärmung kann soweit führen, dass das Gelenk versagt, weil Teile des Gelenkes die Form oder die strukturelle Festigkeit verlieren oder weil die Elektronik außerhalb der erlaubten Betriebsparameter betrieben wird. Der Widerstand wird dabei bevorzugt so verändert, dass die dissipierte Energie verringert wird. Aufgrund der geringeren umzuwandelnden Energiemenge können sich die Widerstandseinrichtung oder andere Komponenten des künstlichen Gelenkes abkühlen und in einem Temperaturbereich arbeiten, für den sie vorgesehen sind. Darüber hinaus kann vorgesehen sein, dass die Widerstandseinrichtung so angepasst wird, dass Veränderungen, die aufgrund einer Temperaturveränderung auftreten, ausgeglichen werden. Verringert sich beispielsweise die Viskosität einer Hydraulikflüssigkeit aufgrund der Erwärmung, kann die Widerstandseinrichtung entsprechend verstellt werden, um weiterhin die gewohnten Flexionswiderstände und Extensionswiderstände bereitzustellen, damit der Prothesen- oder Orthesennutzer weiterhin auf ein ihm bekanntes Verhalten des künstlichen Gelenkes vertrauen kann. Dabei wird die Temperatur der Widerstandseinrichtung gemessen und der Steuerung zugrunde gelegt, alternativ dazu können auch andere Einrichtungen der Temperaturmessung unterzogen werden, wenn diese ein temperaturkritisches Verhalten aufweisen. Ist beispielsweise eine Steuerungselektronik besonders temperaturempfindlich, empfiehlt es sich, diese alternativ oder ergänzend zu der Widerstandseinrichtung zu überwachen und einen entsprechenden Temperatursensor dort vorzusehen. Sind einzelne Komponenten, beispielsweise aufgrund der verwendeten Materialien, temperaturempfindlich, empfiehlt es sich, an den entsprechenden Stellen eine Messeinrichtung vorzusehen, um entsprechende Temperatursignale erhalten zu können.

Sofern das künstliche Gelenk als ein Gelenk der unteren Extremität ausgebildet ist, ist in einer Variante vorgesehen, dass für die Standphase, z.B. während des Gehens, bei zunehmender Temperatur der Widerstand erhöht wird. Dabei kann sowohl der Extensions- als auch der Flexionswiderstand erhöht werden. Durch den erhöhten Widerstand ist der Nutzer gezwungen, langsamer zu gehen und kann somit weniger Energie in das Gelenk einbringen. Dadurch kann das Gelenk abkühlen, so dass es innerhalb der zulässigen Betriebsparameter betrieben werden kann.

Eine weitere Variante beim Einsatz von einem künstlichen Gelenk an einer unteren Extremität sieht vor, dass beim Gehen für die Schwungphase der Beugewiderstand bei zunehmender Temperatur verringert wird. Wird in der oder für die Schwungphase der Beugewiderstand reduziert, führt dies dazu, dass das Gelenk weiter ausschwingt. Der Prothesenfuß gelangt damit später nach vorne zum Fersenauftritt, wodurch der Nutzer wiederum gezwungen wird, langsamer zu gehen, was zu einer reduzierten Energieumwandlung in Wärme führt.

Der Widerstand kann bei Erreichen oder bei Überschreiten eines Temperaturschwellwertes verändert werden. Der Widerstand kann dabei sprungartig bei Erreichen oder Überschreiten einer Temperaturschwelle verändert werden, so dass ein Umschalten des Widerstandswertes bzw. der Widerstandswerte erfolgt. Vorteilhaft ist vorgesehen, dass eine kontinuierliche Veränderung des Widerstandes mit der Temperatur erfolgt, nachdem der Temperaturschwellwert erreicht wird. Wie hoch der Temperaturschwellwert angesetzt wird, liegt an den jeweiligen konstruktiven Parametern, verwendeten Materialien und der angestrebten Gleichmäßigkeit des Widerstandsverhaltens der Prothese oder Orthese. Der Widerstand in der Standphase darf unter anderem nicht so weit erhöht werden, dass dadurch eine sicherheitskritische Situation, z.B. beim Treppabsteigen, erzeugt wird.

Die temperaturinduzierte Widerstandsänderung ist nicht der einzige Steuerungsparameter einer Widerstandsänderung, vielmehr ist vorgesehen, dass eine solche temperaturinduzierte Widerstandsänderung einer funktionalen Widerstandsänderung überlagert wird. Ein künstliches Gelenk, beispielsweise ein Kniegelenk oder ein Knöchelgelenk, wird über eine Vielzahl von Parametern situationsabhängig gesteuert, so dass so genannte funktionale Widerstandsänderungen, die beispielsweise auf der Grundlage der Gehgeschwindigkeit, der Gehsituation oder dergleichen erfolgen, durch die Widerstandsänderung aufgrund der Temperatur ergänzt werden.

Es kann weiterhin vorgesehen sein, dass bei Erreichen oder Überschreiten eines Temperaturschwellwertes ein Warnsignal ausgegeben wird, um dem Nutzer der Prothese oder Orthese bewusst zu machen, dass sich das Gelenk oder die Widerstandseinrichtung in einem kritischen Temperaturbereich befindet. Das Warnsignal kann als ein taktiles, optisches oder akustischen Warnsignal ausgegeben werden. Ebenfalls sind Kombinationen der verschiedenen Ausgabemöglichkeiten vorgesehen.

Es kann eine Einstellvorrichtung vorgesehen sein, über die der Grad der Widerstandsänderung verändert wird. Beispielsweise kann auf der Grundlage ermittelter Daten, beispielsweise des Gewichtes des Nutzers der Orthese oder Prothese oder der ermittelten Axialkraft beim Auftreten, erkannt werden, dass eine überproportional hohe Widerstandsänderung erfolgen muss. Ebenfalls besteht die Möglichkeit, dass eine manuelle Einstellvorrichtung vorgesehen ist, über die eine Anpassung der jeweiligen Widerstandsänderung erfolgt, so dass eine tendenziell größere oder geringere Widerstandsänderung in Abhängigkeit von eingestellten oder ermittelten Daten erfolgen kann.

Die erfindungsgemäße Vorrichtung zur Durchführung des Verfahrens, wie es oben beschrieben ist, sieht vor, dass eine einstellbare Widerstandseinrichtung, die zwischen zwei gelenkig aneinander angeordneten Komponenten eines künstlichen orthetischen oder prothetischen Gelenkes angeordnet ist sowie eine Steuereinrichtung und Sensoren, die Zustandsinformationen in der Vorrichtung erfassen, vorhanden sind. Es sind zumindest ein

Temperatursensor und eine Einstellvorrichtung vorgesehen, wobei über die Einstellvorrichtung eine temperaturabhängige Widerstandsänderung aktivierbar und/oder deaktivierbar ist. Dadurch ist es möglich, eine wahlweise temperaturgesteuerte Widerstandsänderung vorzunehmen und diese besondere Funktion oder Zusatzfunktion z.B. eines Kniesteuerungsverfahrens bewusst zu aktivieren oder zu deaktivieren.

Nachfolgend wird ein Ausführungsbeispiel der Erfindung anhand der beigefügten Figuren näher erläutert. Es zeigen:
- Figur 1 -: das Verhalten von Kenngrößen bei zunehmendem Widerstand in der Standphase;
- Figur 2 -: das Verhalten von Kenngrößen bei fallendem Widerstand in der Schwungphase;
- Figur 3 -: Kniewinkelverlauf und Widerstandskurve beim Gehen in der Ebene; sowie
- Figur 4 -: Kniewinkelverlauf und Widerstandskurve beim Gehen auf einer schiefen Ebene.

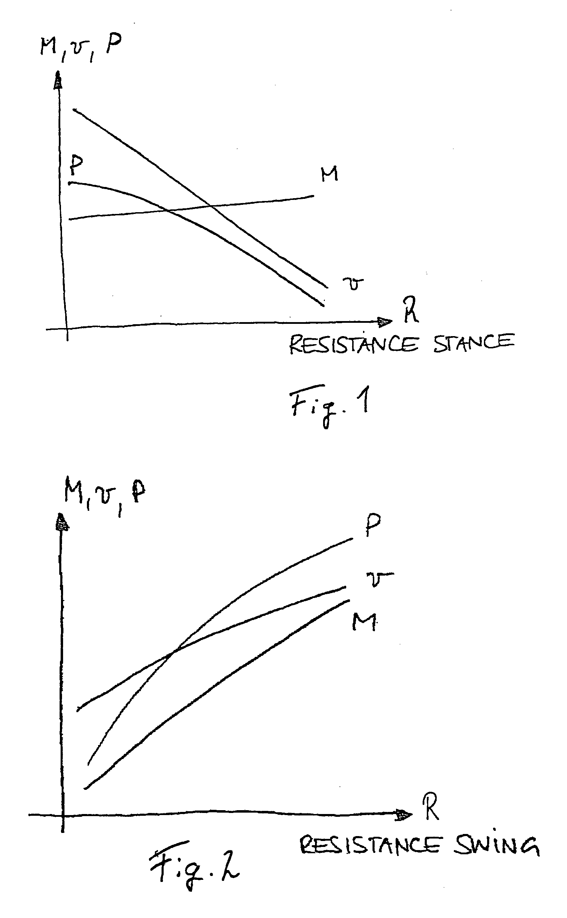

In der Figur 1 ist exemplarisch in dem Diagramm die Abhängigkeit der Kenngrößen Kniemoment M, Leistung P und Geschwindigkeit v über den Widerstand R in der Standphase bei einem Prothesenkniegelenk aufgetragen. In dem Prothesenkniegelenk sind dabei eine Widerstandseinrichtung und ein Aktuator angeordnet, über den der Widerstand, der der Beugung und/oder Streckung entgegengesetzt wird, verändert werden kann. Neben einer Prothese kann auch eine entsprechend ausgestattete Orthese verwendet werden, ebenfalls sind andere Gelenkeinrichtungen als Einsatzgebiet möglich, beispielsweise Hüft- oder Fußgelenke. In der Widerstandseinrichtung wird in der Regel die mechanische Energie in thermische Energie umgewandelt, um die Bewegung eines Unterschenkelteils relativ zu einem Oberschenkelteil abzubremsen, Entsprechendes gilt für andere Gelenke.

Die Temperatur der Widerstandseinrichtung hängt dabei davon ab, wie groß die Leistung P ist, die während der Standphase aufgebracht wird. Die Leistung P hängt von dem wirksamen Kniemoment M und der Geschwindigkeit v ab, mit der das Kniegelenk gebeugt wird. Diese Geschwindigkeit hängt wiederum von dem Widerstand R ab, der durch die nicht dargestellte Widerstandseinrichtung der jeweiligen Bewegung in der Standphase entgegengesetzt wird. Werden - insbesondere beim Abwärtsgehen auf Rampen oder Treppen, wo sich die Widerstandseinrichtung typischerweise am stärksten erwärmt- in der Standphase nach dem Fersenstoß der Flexionswiderstand und im weiteren Verlauf bei einer einsetzenden Extensionsbewegung der Extensionswiderstand erhöht, verringert sich die Bewegungsgeschwindigkeit der Gelenkkomponenten zueinander und damit auch die Bewegungsgeschwindigkeit der Widerstandseinrichtung. Durch die überproportional stärkere Verringerung der Geschwindigkeit v verringert sich die Leistung P während der Standphase trotz steigendem Moment M, sodass die umzuwandelnde Energie entsprechend der sich verringernden Leistung P abnimmt. Dementsprechend verringert sich bei einer gleich bleibenden Kühlung die Temperatur der Widerstandseinrichtung oder derjenigen Komponente, die hinsichtlich ihrer Temperatur überwacht wird.

In der Figur 2 ist die Korrelation der beschriebenen Kenngrößen zu dem Widerstand R in der Schwungphase dargestellt. Bei einer Verringerung des Widerstandes R während der Schwungphase verringern sich die Geschwindigkeit v, das Kniemoment M und damit auch die aufgebrachte Leistung P, so dass die umzuwandelnde Energie verringert wird. Dementsprechend verringert sich die Temperatur der Widerstandseinrichtung bei einem abnehmenden Schwungphasenwiderstand.

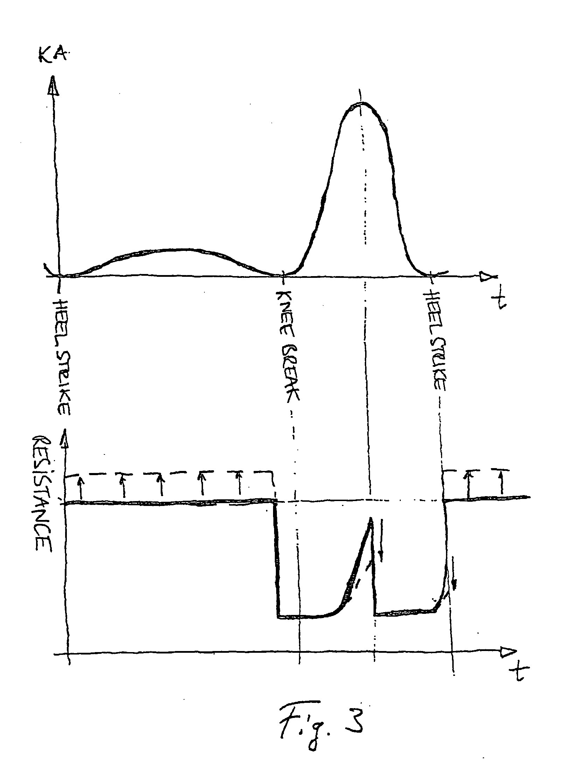

Die Figur 3 zeigt in dem oberen Diagramm den Kniewinkel KA über die Zeit t, beginnend mit dem so genannten "heel strike", dem Fersenstoß, der in der Regel bei einem gestreckten Kniegelenk ausgeführt wird. Während des Aufsetzens des Fußes erfolgt eine geringe Flexion des Kniegelenkes, die so genannte Standphasenflexion, um das Aufsetzen des Fußes und den Fersenstoß abzumildern. Nachdem der Fuß vollständig aufgesetzt ist, wird das Knie vollständig gestreckt, bis zum so genannten "knee break", bei dem das Kniegelenk eingebeugt wird, um das Kniegelenk nach vorne zu bewegen und über den Vorderfuß abzurollen. Von dem "knee break" ausgehend erhöht sich der Kniewinkel KA bis zum maximalen Kniewinkel in der Schwungphase, um dann wieder nach dem Vorbringen des gebeugten Beines und des Prothesenfußes in eine Streckstellung überzugehen, und dann wieder mit der Ferse aufzusetzen. Dieser Kniewinkelverlauf ist typisch für das Gehen in der Ebene.

In der unteren Grafik ist der Widerstand R über die Zeit aufgetragen, korrespondierend zu dem entsprechenden Kniewinkel. Ob ein Extensions- oder Flexionswiderstand vorliegt, hängt von dem Kniewinkelverlauf ab, bei zunehmendem Kniewinkel ist der Flexionswiderstand wirksam, bei abnehmendem Kniewinkel der Extensionswiderstand. Nach dem "heel strike" ist ein relativ hoher Flexionswiderstand vorhanden; nach der Bewegungsumkehr ein hoher Extensionswiderstand. Bei "knee break" wird der Widerstand reduziert, um da Einbeugen und Vorbringen des Knies zu erleichtern. Dadurch wird das Gehen erleichtert. Nach dem Absenken des Widerstandes bei "knee break" wird der Widerstand über einen Teil der Schwungphase auf dem niedrigen Niveau gehalten, um ein nach hinten Schwingen des Prothesenfußes zu erleichtern. Um die Schwungbewegung nicht zu groß werden zu lassen, wird vor Erreichen des Kniewinkelmaximums der Flexionswiderstand erhöht und nach Erreichen des Kniewinkelmaximums und der Bewegungsumkehr der Extensionswiderstand auf das niedrige Niveau der Schwungphasenbeugung verringert. Die Widerstandsverringerung des Extensionswiderstandes bleibt auch über die Extensionsbewegung in der Schwungphase erhalten, bis kurz vor dem "heel strike". Kurz vor Erreichen der vollständigen Streckung wird der Widerstand erneut erhöht, um ein hartes Anschlagen in den Streckanschlag zu vermeiden. Um beim Aufsetzen des Prothesenfußes eine ausreichende Sicherheit gegen ein unkontrolliertes Einknicken zu erhalten, ist auch der Flexionswiderstand auf einem hohen Niveau.

Wird nun der Flexionswiderstand erhöht, was durch die gestrichelte Linie angedeutet ist, verlangsamt sich die Kniewinkelgeschwindigkeit und damit auch das Gehen des Prothesennutzers. Nach dem "heel strike" folgt nur eine vergleichsweise geringe Beugung in der Standphasenflexion und eine langsame Streckung, was die dissipierte Energie geringer macht. Das Anheben des Flexionswiderstandes vor Erreichen des Kniewinkelmaximums erfolgt in einer weniger ausgeprägten Weise als bei der Standard-Dämpfung, was durch den nach unten gerichteten Pfeil angedeutet ist. Dadurch schwingt der Unterschenkel und damit der Prothesenfuß weiter aus, so dass ein größerer Zeitraum zwischen den "heel strikes" vorliegt. Auch das Verringern des Flexionswiderstandes in der Schwungphasenflexion führt zu einer Verringerung der Gehgeschwindigkeit.

Am Ende der Schwungphasenextension, also kurz vor dem Auftreten und dem "heel strike", wird der Extensionswiderstand im Vergleich zu dem Standard-Niveau verringert. Es ist also vorgesehen, dass der Extensionswiderstand verringert wird, so dass das Unterschenkelteil schneller in die Streckung gelangt, so dass sich die Leistung P und damit die zu dissipierende Energie verringert. Während der Standphase zwischen dem "heel strike" und dem "knee break" können sowohl der Flexionswiderstand als auch der Extensionswiderstand erhöht werden, um das Einbeugen während der Standphase zu vermindern und damit die dissipierte Energie zu reduzieren.

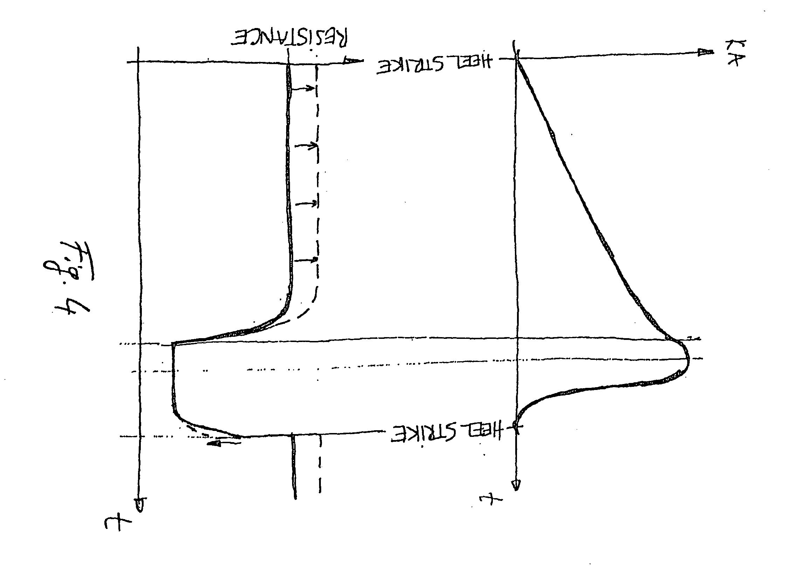

In der Figur 4 ist in der oberen Darstellung der Kniewinkelverlauf beim Gehen auf einer Rampe gezeigt, hier auf einer abschüssigen Rampe. Nach dem "heel strike" erfolgt eine kontinuierliche Vergrößerung des Kniewinkels KA bis zum Kniewinkelmaximum. Dieser Winkelverlauf entsteht bei alternierendem Rampabgehen. Das Knie übernimmt das Gewicht des Trägers, sinkt langsam ein und entlastet somit die kontralaterale Seite. Nach dem Erreichen des Kniewinkelmaximums erfolgt ein schnelles Vorbringen des Knies und des Unterschenkels bis zu einer vollständigen Streckung, die mit dem erneuten "heel strike" einhergeht. Der Flexionswiderstand bleibt dabei über einen weiten Verlauf auf einem konstant hohen Niveau, bis er dann abgesenkt wird, um ein weites Einbeugen des Knies und damit ein Abheben des Prothesenfußes und ein Zurückschwingen zu ermöglichen. Dieses Zurückschwingen findet nach Erreichen des Minimums des Widerstandes bis zum Erreichen des Kniewinkelmaximums statt. Anschließend wird auf einem niedrigen Widerstandsniveau der Extensionswiderstand beibehalten, bis er kurz vor dem Auftreten erneut angehoben wird.

Liegen nun erhöhte Temperaturen in der Widerstandseinrichtung vor, werden die Widerstände in der Standphase erhöht, um eine langsame Gehgeschwindigkeit und ein langsames Einbeugen zu gewährleisten. Nach Erreichen der Schwungphase und dem nach vorne Bringen des Prothesenfußes wird auch der Extensionswiderstand verringert, was ebenfalls zu einer Verringerung der zu dissipierenden Energie führt.

## Patentansprüche

1. Verfahren zur Steuerung eines künstlichen orthetischen oder prothetischen Gelenkes mit einer Widerstandseinrichtung, der zumindest ein Aktuator zugeordnet ist, über den der Beuge- und/oder Streckwiderstand (R) in Abhängigkeit von Sensordaten verändert wird, **dadurch gekennzeichnet, dass** die Temperatur der Widerstandseinrichtung gemessen und der Steuerung zu Grunde gelegt wird.

2. Verfahren nach Anspruch 1, **dadurch gekennzeichnet, dass** das künstliche Gelenk als ein Gelenk einer unteren Extremität ausgebildet ist und während der Standphase bei zunehmender Temperatur der Widerstand (R) erhöht wird.

3. Verfahren nach Anspruch 1 oder 2, **dadurch gekennzeichnet, dass** das künstliche Gelenk als ein Gelenk einer unteren Extremität ausgebildet ist und während der Schwungphase der Beugewiderstand bei zunehmender Temperatur verringert wird.

4. Verfahren nach einem der voranstehenden Ansprüche, **dadurch gekennzeichnet, dass** der Widerstand (R) bei Erreichen oder Überschreiten eines Temperaturschwellwertes verändert wird.

5. Verfahren nach einem der voranstehenden Ansprüche, **dadurch gekennzeichnet, dass** der Widerstand (R) in Abhängigkeit von zumindest einem gemessenen Temperatursignal kontinuierliche mit der sich verändernden Temperatur verändert wird.

6. Verfahren nach einem der voranstehenden Ansprüche, **dadurch gekennzeichnet, dass** die temperaturinduzierte Widerstandsänderung einer funktionellen Widerstandsänderung überlagert wird.

7. Verfahren nach einem der voranstehenden Ansprüche, **dadurch gekennzeichnet, dass** bei Erreichen oder Überschreiten eines Temperaturschwellwertes ein Warnsignal ausgegeben wird.

8. Verfahren nach einem der voranstehenden Ansprüche, **dadurch gekennzeichnet, dass** eine Einstellvorrichtung vorgesehen ist, über die der Grad der Widerstandsänderung verändert wird.

9. Vorrichtung zur Durchführung des Verfahren nach einem der voranstehenden Ansprüche, mit einer einstellbaren Widerstandseinrichtung, die zwischen zwei gelenkig aneinander gelagerten Komponenten eines künstlichen orthetischen oder prothetischen Gelenkes angeordnet ist, mit einer Steuereinrichtung und Sensoren, die Zustandsinformationen der Vorrichtung erfassen, **dadurch gekennzeichnet, dass** zumindest ein Temperatursensor und eine Einstellvorrichtung vorgesehen sind und dass über die Einstellvorrichtung eine temperaturabhängige Widerstandsänderung aktivierbar und/oder deaktivierbar ist.

## Claims

1. A method for controlling an artificial orthotic or prosthetic joint with a resistance device to which at least one actuator is assigned, via which actuator the flexion and/or extension resistance (R) is changed depending on sensor data, **characterized in that** the temperature of the resistance device is measured and used as a basis for the control.

2. The method as claimed in claim 1, **characterized in that** the artificial joint is designed as a joint of a lower extremity, and the resistance (R) is increased during the stance phase with rising temperature.

3. The method as claimed in claim 1 or 2, **characterized in that** the artificial joint is designed as a joint of a lower extremity, and the flexion resistance is reduced during the swing phase with rising temperature.

4. The method as claimed in one of the preceding claims, **characterized in that** the resistance (R) is changed when a temperature threshold value is reached or exceeded.

5. The method as claimed in one of the preceding claims, **characterized in that** the resistance (R) is changed continuously with the changing temperature.

6. The method as claimed in one of the preceding claims, **characterized in that** the temperature-induced change of resistance superposes a functional change of resistance.

7. The method as claimed in one of the preceding claims, **characterized in that** a warning signal is output when a temperature threshold value is reached or exceeded.

8. The method as claimed in one of the preceding claims, **characterized in that** an adjustment device is provided, via which the degree of the change of resistance is changed.

9. An appliance for carrying out the method as claimed in one of the preceding claims, with an adjustable resistance device, which is arranged between two mutually articulated components of an artificial orthotic or prosthetic joint, and with a control device and sensors, which detect status information of the appliance, **characterized in that** at least one temperature sensor and an adjustment device are provided, and **in that** a temperature-dependent change of resistance can be activated and/or deactivated via the adjustment device.

## Revendications

1. Procédé pour commander une articulation orthétique ou prothétique artificielle avec un système à résistance auquel est associé au moins un actionneur au moyen duquel on modifie la résistance au pliage et/ou à l'extension (R) en fonction de données sensorielles, **caractérisé en ce que** la température du système à résistance est mesurée et est mise à la base de la commande.

2. Procédé selon la revendication 1, **caractérisé en ce que** l'articulation artificielle est réalisée comme une articulation d'une extrémité inférieure et on augmente la résistance (R) pendant la phase en station debout lorsque la température augmente.

3. Procédé selon la revendication 1 ou 2, **caractérisé en ce que** l'articulation artificielle est réalisée comme une articulation d'une extrémité inférieure et on réduit la résistance au pliage pendant la phase d'élan lorsque la température augmente.

4. Procédé selon l'une des revendications précédentes, **caractérisé en ce que** l'on modifie la résistance (R) lorsqu'on atteint ou on dépasse une valeur seuil de température.

5. Procédé selon l'une des revendications précédentes, **caractérisé en ce que** l'on modifie la résistance (R) en continu avec la température qui varie en fonction d'au moins un signal de température mesuré.

6. Procédé selon l'une des revendications précédentes, **caractérisé en ce que** la variation de résistance induite par la température est superposée d'une variation de résistance fonctionnelle.

7. Procédé selon l'une des revendications précédentes, **caractérisé en ce que** l'on délivre un signal d'avertissement en cas d'atteinte ou de dépassement d'une valeur seuil de température.

8. Procédé selon l'une des revendications précédentes, **caractérisé en ce qu'**il est prévu un dispositif de réglage au moyen duquel on modifie le degré de variation de résistance.

9. Appareil pour la mise en oeuvre du procédé selon l'une des revendications précédentes, comprenant un système à résistance réglable qui est agencé entre deux composantes, disposées de façon articulée l'une par rapport à l'autre, d'une articulation orthétique ou prothétique artificielle, comprenant un système de commande et des capteurs qui détectent des informations d'état de l'appareil, **caractérisé en ce qu'**il est prévu au moins un capteur de température et un dispositif de réglage, et **en ce qu'**il est possible d'activer et/ou de désactiver une variation de résistance en dépendance de la température via le dispositif de réglage.
